# EUROPEAN PATENT APPLICATION

(11) **EP 3 524 625 A1**
(43) Date of publication of application: **14.08.2019**
(21) Application number: 18155716.6
(22) Date of filing: 08.02.2018
(51) Int. Cl.: C07K 16/28

(54) **ANTIBODY AGAINST ALPHA-11 INTEGRIN AND ITS USE**

(71) Applicant: Bergen Teknologioverføring AS, 5006 Bergen (NO)
(72) Inventor: Gullberg, Elon Donald, 5006 Bergen (NO)
(74) Representative: Gramm, Lins & Partner Patent- und Rechtsanwälte PartGmbB

(57) **Abstract**

In a first aspect, the present invention relates to an antibody directed against the alpha-11 integrin subunit, in particular, said antibody is an antibody binding the same epitope as the antibody 203 E1H5 produced by the hybridoma deposited as DSM ACC3318 or binding to the same epitope of the alpha-11 integrin subunit as bound by 234 H11E8 produced by the hybridoma deposited as DSM ACC3319. Further, the present invention relates to a pharmaceutical composition containing the antibodies according to the present invention as well as the use of the antibody or the pharmaceutical composition, accordingly, in treating or preventing fibrosis, cancer, sclero-derma or excessive scarring observed in hypertrophic scars and keloids. Finally, the present invention relates to a kit for treating or preventing cancer, fibrosis or sclero-derma as well as for preventing excessive scarring in wound healing comprising the antibody according to the present invention or the pharmaceutical according to the present invention.

## Description

In a first aspect, the present invention relates to an antibody directed against the alpha-11 integrin subunit, in particular, said antibody is an antibody binding the same epitope as the antibody 203 E1 H5 produced by the hybridoma deposited as DSM ACC3318 or binding to the same epitope of the alpha-11 integrin subunit as bound by 234 H11 E8 produced by the hybridoma deposited as DSM ACC3319. Further, the present invention relates to a pharmaceutical composition containing the antibodies according to the present invention as well as the use of the antibody or the pharmaceutical composition, accordingly, in treating or preventing fibrosis, cancer, scleroderma or excessive scarring observed in hypertrophic scars and keloids. Finally, the present invention relates to a kit for treating or preventing cancer, fibrosis or scleroderma as well as for preventing excessive scarring in wound healing comprising the antibody according to the present invention or the pharmaceutical according to the present invention.

### Prior art

Integrins are heterodimeric cell surface receptors composed of non-covalently associated alpha and beta subunits, which act as cell surface links to extracellular matrix (ECM) and to other cells in dynamic cell-cell linkages.

Integrin subunits are composed of different domains with different functions. The extracellular domain of collagen-binding alpha integrin chains contain an inserted alpha I domain, which is responsible for collagen-binding without direct involvement of the beta subunit. Whereas different integrin alpha chains display conserved regions including their cytoplasmic tail, the cytoplasmic tails of integrin alpha chains show little sequence similarity except for the vary proximal membrane sequence GFFXR. Of note, alpha-11 integrin lacks this conserved sequence but contains the GFFRS sequence. This conserved sequence is integral for the activity of the integrin. Namely, mutations of this sequence in general result in integrins being constitutively active.

Integrins are involved in various functions including various signal transduction pathways mediating cellular signals. These signal transduction pathways include the regulation of the cell cycle, the organization of the intracellular cytoskeleton, the movement of new receptors to the cell membrane, etc. The presence of integrins allows rapid and flexible responses to events at the surface. The integrin alpha-11 is a polypeptide that is encoded in humans by the ITGA11 gene. It is described that the integrin alpha-11 binds collagen when present as a heterodimer with beta-1 integrin. Alpha-11 beta-1 integrin represents a collagen receptor, which is the latest identified member of the vertebrate integrin family. This integrin heterodimer has distinct functions *in vivo* from other collagen-binding integrins. It was demonstrated in mice that collagen-binding integrins are dispensable for normal development, but suggest important roles in tissue remodeling events occurring in wound healing, fibrosis and tumor-stroma interactions.

For example, it is described that non-small lung cancers (NSCLC) express alpha-11 integrin subunit in the activated stroma, when it has the potential to be a biomarker for activated cancer associated fibroblasts [1-3].

Alpha-11 integrin has been shown to have a pro-fibrotic role in diabetic cardiomyopathy, a condition in which high levels of glucose lead to the glycation of collagen, resulting in heart fibrosis. In an experimental rat disease model, the interaction of cardiac fibroblasts with glycated collagen through alpha-11 beta-1 increased TGF-β2 expression, which in turn induced αSMA expression. The increased level of alpha-11 subunit under these conditions has been interpreted as an attempt by the cells to compensate for reduced adhesion. In the rat diabetes model, TGF-β2 is responsible for the alpha-11 beta-1 increased expression.

In Tumorigeneses, an important role for alpha-11 beta-1 has been described already in the beginning of 2000 where this integrin was identified as a novel candidate biomarker gene for the activated stroma in non-small-cell lung cancer. Further, a role for alpha-11 beta-1 integrin in tumorigeneses was indicated in xenograft experiments where the mixing of tumor cells with alpha-11 expressing fibroblasts was shown to stimulate tumor growth. It was speculated that alpha-11 expressing fibroblasts stimulate the autocrine secretion of CXCL5 in said NSCLC cells. In a separate study breast cancer cells , in a model for tumor invasion, expressed alpha11 at the invasive front and was found to be part of a 7 gene signature of invasive, mesenchymal trail-blazer cells[4]. That is, alpha-11 beta-1 integrin has been reported to be upregulated in some tumor forms. However, the exact role of beta-11 in the tumor stroma during TGF-β dependent myofibroblast differentiation, tumor growth, and tumor metastasis remains to be elucidated.

The importance of the tumor microenvironment for tumor growth and tumor spread is increasingly being recognized. The major cell types in the tumor stroma of solid tumors include cancer-associated fibroblasts (CAFs) of varying origin, endothelial cells, pericytes, mesenchymal stem cells, tumor stem cells and immune cells. The ECM in addition to serving as a structural scaffold serves as a reservoir of growth factors and cytokines, which take part in bidirectional communication that occurs between the stroma and the tumor cells. For example, CAFs produce collagen crosslinking enzymes of the LOX family, which can increase stiffness of ECM, affecting the growth and invasion of tumor cells. CAFs thus constitute a group of fibroblastic cells of different origin, some of which share characteristics with myofibroblasts in granulation tissue during wound healing and tissue fibrosis. The mesenchymally derived CAF population seems to represent a heterogeneous cell mixture compared to resident tissue fibroblasts in typical resting tissue. For contractile activated myofibroblasts and CAFs the protein alpha smooth muscle actin (alpha SMA) has been suggested as a suitable marker. However, careful analysis of collagen producing activated fibroblast in heart, lung and kidney fibrosis has revealed that only a fraction of these fibroblasts expressed alpha SMA suggesting that alpha SMA is an inconsistent marker of activated collagen producing cells [5]. There is thus a need for better cell type specific markers to be able to understand the dynamics of different CAF population in the tumor stroma. As noted, NSCLC stroma express integrin alpha-11 and, in addition, recent studies suggest that the stroma expression of alpha-11 in NSCLC correlates with LOXL1 expression and tumor stiffness. In fibrosis models importance of integrins have been demonstrated. Although expression of alpha-11 integrin in NSCLC in the activated stroma has been described, and regulation of cancer stroma stiffness of said integrin as well as promoting tumorgenicity and metastases, is disclosed. A suitable active agent for treating or preventing said above-mentioned diseases is lacking.

Moreover, suitable markers to discriminate between different subclasses of fibroblasts are lacking in tissue fibrosis, scleroderma, excessive scarring conditions, joint disease and cancer.

### Brief description of the present invention

Hence, an object of the present invention is the provision of active agents allowing to target the alpha-11 integrin subunit for use in treating, fibrosis scleroderma, excessive scarring, joint disease as well as cancer. In addition, an object of the present invention is the provision of a marker suitable for determining alpha-11 integrin subunit in samples, in particular, tissue samples, like tissue samples obtained from fibrosis and cancer patients.

The present inventors aim in providing an antibody which allows functional blocking of alpha-11 or which represents a suitable antibody allowing determining the alpha-11 integrin subunit in cells and tissues. For determining the alpha-11 integrin subunit, the marker antibody according to the present invention allows to detect said subunit in fixed samples, in particular, in acetone-fixed or formaldehyde-fixed samples, e.g. in samples being cryopreserved.

In a first aspect, the present invention provides an antibody i) that binds to the same epitope of the alpha-11 integrin subunit as bound by 203 E1 H5 produced by the hybridoma deposited as DSM ACC3318 or ii) that binds the same epitope of the alpha-11 integrin subunit as bound by 234 H11 E8 produced by the hybridoma deposited as DSM ACC3319.

In an embodiment, the antibody is either 203 E1 H5 produced by the hybridoma deposited as DSM ACC3318 or 234 H11 E8 produced by the hybridoma deposited as DSM ACC3319. In a further embodiment, the antibody is a humanized antibody.

In a further aspect, the present invention relates to a pharmaceutical composition containing the antibody according to the present invention, and, optionally, a pharmaceutically acceptable excipient, carrier or diluent.

In a further embodiment, the present invention relates to the use of the antibody or the pharmaceutical composition according to the present invention in treating or preventing cancer, in treating or preventing fibrosis, in treating or preventing scleroderma or in preventing excessive scarring in wound healing, e.g. observed in hypertrophic scars an keloids.

Further, the present invention relates to a kit for treating or preventing cancer, fibrosis or scleroderma as well as for preventing excessive scarring in wound healing comprising the antibody according to the present invention or the pharmaceutical composition according to the present invention, optionally, with a further therapeutic agent for treating said disease.

In a further aspect, the present invention relates to a method for preventing or treating cancer as well as fibrosis or scleroderma or for preventing excessive scarring in wound healing, e.g. observed in hypertrophic scars an keloids including the step of administering an antibody according to the present invention or a pharmaceutical composition according to the present invention to a subject in need thereof.

Moreover, the present invention relates to the use of the antibodies according to the present invention as well as methods using the antibodies according to the present invention for purifying, in particular, for isolating mesenchymal stem cells from a cell population. These isolated or purified mesenchymal stem cells may be used in various applications including production of artificial tissue, etc. In addition, the methods and uses allow to separate fibroblasts from other cell types, thus, allowing cartilage cell cultures with cartilage cells being alpha-11 negative but alpha-10/beta-1 positive.

Finally, the present invention provides molecules being antibody drug conjugates which are suitable for pharmaceutical purposes. In particular, said antibody drug conjugates contain antibodies being humanized by known methods.

### Brief description of the drawings

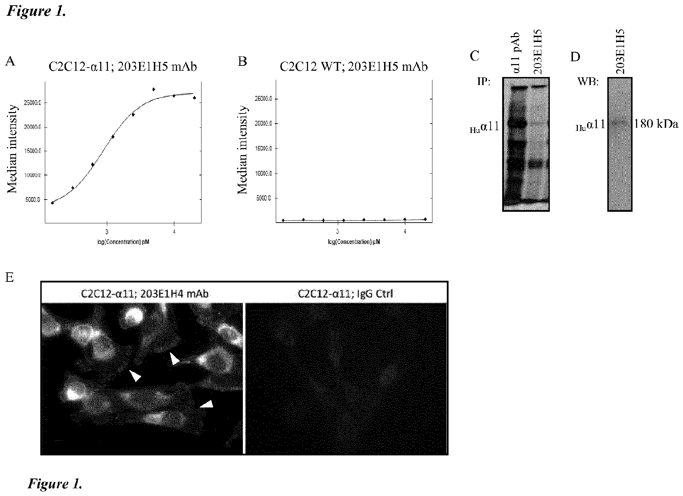

Figure 1. Immunological characterization of 203E1H5 antibody A-B. C2C12 mouse myoblast cells expressing human integrin α11-EGFP or WT (wildtype) C2C12 cells were stained with 203E1 H5 monoclonal antibody (mAb) for 1 hour at 4°C. Cells were then washed and stained with goat anti-mouse R-Phyco-erythrin IgG. Samples were analysed by flow cytometry using Intellicyt iQue and fluorescence intensity was measured. C. Metabolic labelling of proteins and immunoprecipitation (IP) of integrin α11β1 with the 203E1 H5 mAb and the polyclonal α11 antibody (α11 polyclonal antibody(pAb)). Protein bands of integrin chain α11 and β1 migrated at the expected size after precipitation using α11 antibodies. D. Western-blotting (WB) using the α11 203E1 H5 mAb on C2C12-hu α11-EGFP lysate, shown is the protein band of expected size of α11-EGFP (180 kDa). E. C2C12 mouse myoblast cells expressing human integrin α11 were allowed to attach on collagen I- cocated coverslips for 2 hours, cells were then fixed with methanol and stained with 203E1 H5 mAb or control IgG. Integrin α11 staining in cell focal adhesions is indicated by white triangles.

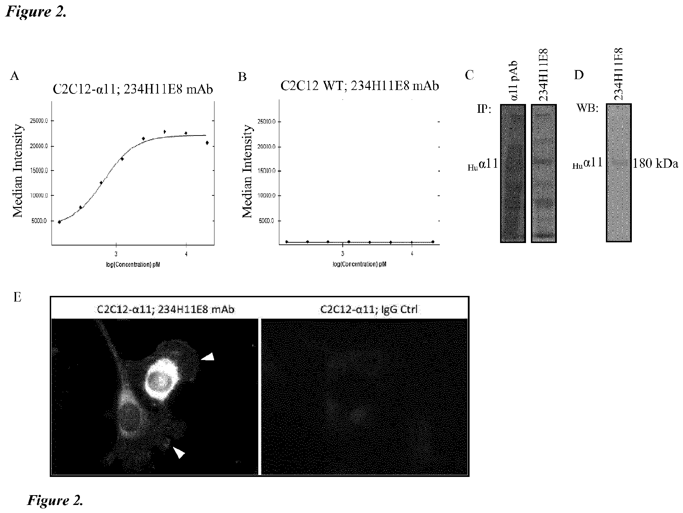

Figure 2. Immunological characterization of 234H11E8 antibody A-B. C2C12 mouse myoblast cells expressing human integrin α11-EGFP or WT (wildtype) C2C12 cells were stained with 234H11 E8 monoclonal antibody (mAb) for 1 hour at 4°C. Cells were then washed and stained with goat anti-mouse R-Phyco-erythrin IgG. Samples were analyzed by flow cytometry using Intellicyt iQue and fluorescence intensity was measured. C. Metabolic labelling of proteins and immunoprecipitation (IP) of integrin α11β1 with the 234H11 E8 mAb and the polyclonal α11 antibody (α11 pAb). Protein bands of integrin chain α11 and β1 migrated at the expected size after precipitation using α11 antibodies. D. Western-blotting (WB) using the α11 234H11E8 mAb on C2C12-hu α11-EGFP lysate, shown is the protein band of expected size of α11-EGFP (180 kDa). E. C2C12 mouse myoblast cells expressing human integrin α11 were allowed to attach on collagen I- coated coverslips for 2 hours, cells were then fixed with methanol and stained with 234H11E8 mAb or control IgG. Integrin α11 staining in cell focal adhesions was indicated by white triangles.

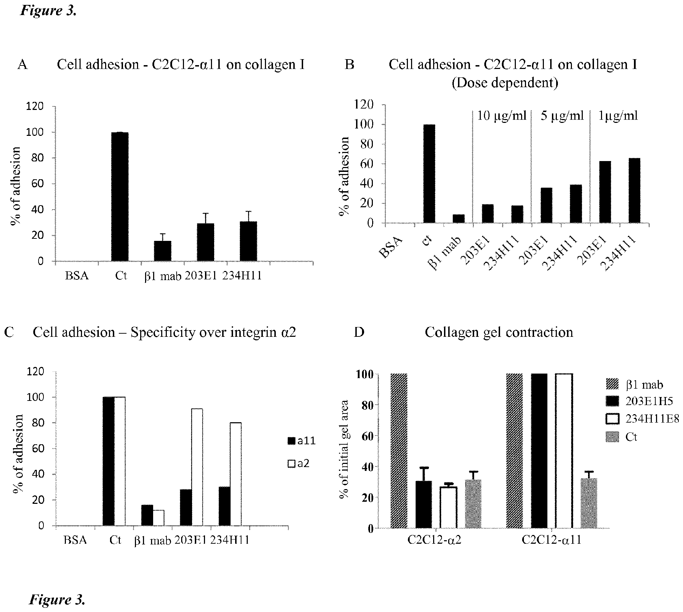

Figure 3. Integrin α11β1 function-blocking by 203E1 H5 and 234H11E8 antobidy A. 203E1 H5 and 234H11E8 mAbs block integrin α11β1 mediated cell adhesion to collagen I. C2C12 mouse myoblast cells expressing human integrin α11(C2C12-α11) were treated with b1 mab or 203E1 H5 or 234H11E8 and allowed to adhere to collagen I. B. 203E1 H5 and 234H11 E8 mAbs block integrin α11β1 mediated cell adhesion to collagen I in a dose dependent manner. C2C12-α11 cells were treated with different concentrations of 203E1 H5 or 234H11E8 and allowed to adhere on collage type I. C. 203E1 H5 and 234H11E8 mAbs do not affect integrin α2β1 mediated cell adhesion to collagen I. C2C12 cells expressing human integrin α2 (C2C12-α2) and C2C12-α11 cells were treated with β1 mab or 203E1 H5 or 234H11E8 and allowed to adhere to collagen I. Untreated cells (Ct) and BSA coated wells were used as controls. Attached cells were fixed with methanol and stained using 0.1 % crystal violet. The percentage of attached cells were measured. D. 203E1 H5 and 234H11E8 mAbs block integrin α11β1-mediated collagen reorganization. C2C12-α2 and C2C12-α11 cells were mixe with neutralized collagen I. Polymerized cell-collagen gels were allowed to float. ß1 mAb or 203E1 H5 or 234H11 E8 mAb were added and the percentage of gel contraction was measured.

### Detailed description of the present invention

The present inventions aims to provide new antibodies suitable for specifically binding the alpha-11 integrin subunit. Namely, the present invention provides antibodies i) that bind to the same epitope of the alpha-11 integrin subunit as bound by 203 E1 H5 produced by the hybridoma deposited as DSM ACC3318 or ii) that bind the same epitope of the alpha-11 integrin subunit as bound by 234 H11 E8 produced by the hybridoma deposited as DSM ACC3319. The antibodies according to the present invention represent functional blocking antibodies blocking e.g. the cell adhesion to collagen I as well as collagen gel reorganization.

Further, the antibodies according to the present invention are suitable for immune detection of the antigen, namely, the alpha-11 integrin subunit, in the samples, in particular, in cryopreserved samples.

As used herein, the term "comprise" or "comprising" as well as "contain" or "containing" include the embodiment of "consist" and "consisting of".

Further, the term "antibody" refers to a polypeptide encoded by an immunoglobulin gene or functional fragments thereof that specifically binds and recognizes an antigen. The recognized immunoglobulin genes include the kappa, lambda, alpha, gamma, delta, epsilon and mu constant region genes, as well as the myriad immunoglobulin variable region genes. Light chains are classified as either kappa or lambda. Heavy chains are classified as gamma, mu, alpha, delta or epsilon, which in turn define the immunoglobulin classes, IgG, IgM, IgA, IgD and IgE, respectively.

The immunoglobulin (antibody) structure unit comprises typically a tetramer composed of two identical pairs of polypeptide chains, each pair having one light and one heavy chain. The N-terminus of each chain defines a variable region of about 100 to 110 or more amino acids primarily responsible for antigen recognition. Thus, the terms "variable heavy chain", V_{H}, or VH, refer to the variable region of an immunoglobulin heavy chain including an Fv, scFv, dsFv or Fab, while the terms "variable light chain", "V_{L}" or "vL" refers to the variable region of the immunoglobulin light chain, including of an Fv, scFv, dsFv or Fab.

Examples of antibody functional fragments include but are not limited to complete antibody molecules, antibody fragments, such as Fv, single chain Fv (scFv), complementarity determining regions (CDRs), VL (light chain variable region), VH (heavy chain variable region), Fab, F(ab)2' and any combination of those or any other functional portion of an immunoglobulin peptide capable of binding to target antigen. The respective antibody and antibody fragments can be obtained by variety of methods known to the skilled person, namely, either by digestion of an intact antibody with an enzyme or by de novo synthesis. The *de novo* synthesis can either be chemically or by using recombinant DNA technology. Unless otherwise indicated, the term antibody includes antibody fragments obtained by the methods known in the art also including phage display libraries. The term "antibody" also includes bivalent or bispecific molecules, diabodies, triabodies and tetrabodies etc.

The term "humanized" antibody is an antibody that retains the reactivity of a non-human antibody while being less immunogenic in humans. This can be achieved by methods along known in the art by retaining the non-human CDR regions and replacing the remaining parts of the antibodies with the human counterparts, a technic well known to the skilled person.

"Single chain Fv (scFv)" or "single chain antibodies" refers to a protein wherein the V_{H} and the V_{L} regions of a scFv antibody comprise a single chain which is folded to create an antigen binding site similar to that found in two chain antibodies. Methods for producing the same are described in the art. Single chain Fv antibodies optionally include a peptide linker of no more than 50 amino acids in length.

The phrase "specifically (or selectively) binds to an antibody" when referring to a protein or peptide, refers to a binding reaction which is determinative of the presence of the protein in the presence of a heterogeneous population of proteins and other biologics. That is, in the designated immunoassay conditions typically present in said immunoassays, the specific antibodies bind to a particular protein, in the present case, the antibody according to the present invention to the alpha-11 integrin subunit and do not bind in a significant amount to other proteins present in the sample. Typically, a specific or selective reaction will be at least twice the background signal or noise and more typically more than 10 to 100 times over the background.

The antibody which is in a one embodiment a monoclonal antibody according to the present invention can be obtained from any animal or the human being, whereby the monoclonal antibody from a mouse are preferred. Further, the monoclonal antibody may be altered biochemically, by genetic manipulation, or it may be synthetic, with the antibody possibly lacking portions completely or in parts, said portions being necessary for the recognition of alpha-11 integrin subunit and being substituted by others imparting further advantageous properties to the antibody.

A hybridoma cell line producing a preferred antibody of the present invention, namely, the monoclonal mouse antibody 203 E1 H5, was deposited at the Deutsche Sammlung von Mikroorganismen und Zellkulturen GmbH (DSMZ) in Braunschweig under the number DSM ACC3318 on March 21, 2017. The hybridoma cell line producing the preferred antibody of the present invention, namely, the monoclonal mouse antibody 234 H11 E8 was deposited at the Deutsche Sammlung von Mikroorganismen und Zellkulturen GmbH (DSMZ) in Braunschweig under the number DSM ACC3319 on March 21, 2017.

The term "epitope" also known as "antigenic determinant" is a part of an antigen that is recognized by the immune system in the present context by antibodies. The epitopes according to the present invention may be conformational epitopes and linear epitopes, based on their structure and interaction with the paratope, namely the part of the antibody that binds to the epitope.

The skilled person is well aware of suitable methods to determine whether an antibody binds to the same epitope of the alpha-11 integrin subunit as bound by 203 E1 H5 produced by the hybridoma deposited as DSM ACC3318 or binds to the same epitope of the alpha-11 integrin subunit as bound by 234 H11 E8 produced by the hybridoma deposited as DSM ACC3319. For example, appropriate tests may include experiments were competitive binding assays of the antibody produced by the hybridoma identified herein and the antibody in question are conducted.

An agent that specifically competes for binding reduces the specific binding of an antibody to a polypeptide, namely, binding to the antigen or paratope. That is, competitive binding assays may be conducted to determine appropriate antibodies using the 203 E1 H5 or 234 H11 E8 antibody produced by the hybridoma cell lines disclosed herewith.

The term "sample" is intended to cover all types of samples suitable for the purpose of the invention. Examples of such samples are serum, sputum, urine, liquor, tissue and biopsies. In particular, the sample may be a blood sample or a tissue sample. In an embodiment, the tissue sample is a paraffin embedded tissue sample, a cryopreserved sample or a cell sample.

In an embodiment, the humanized antibody is an antibody that specifically binds to the alpha-11 integrin subunit, comprising the heavy and light chain complementarity determining regions (CDR) of the antibody according to the present invention. In an embodiment, the CDR are the CDR of the monoclonal antibody produced by the hybridoma deposited as DSM ACC3318 or the CDR of the monoclonal antibody produced by the hybridoma deposited as DSM ACC3319.

As noted above, the antibody according to the present invention, like the monoclonal antibody according to the present invention, are humanized antibodies. That is, the antibody is a humanized antibody, i.e. an antibody that retains the reactivity of a non-human antibody while being less immunogenic in humans. This can be achieved for instance by retaining the non-human CDR regions and replacing the remaining parts of the antibody with the human counterparts. This is a well-established procedure known in the art. In cases where the transfer of the CDR to a human framework leads to a loss of specificity for humanized antibody, back-mutations can be introduced to the framework regions of the human portion of the antibodies. These methods are described in the art accordingly.

In another embodiment, the present invention relates to antibodies, in particular, monoclonal antibodies being selected from the group consisting of scFv, Fab, (Fab')₂.

The antibody according to the present invention, in particular, the monoclonal antibody, like the antibodies being humanized antibodies, may be useful as active agents in pharmaceuticals. That is, the active agents may be used to treat diseases, disorders or conditions where the alpha-11 integrin subunit expression is altered or when alteration of the alpha-11 subunit expression is beneficial to the subject receiving said treatment. Examples of diseases, disorders and conditions include cancer, fibrosis, scleroderma as well as wound healing, namely preventing excessive scarring in wound healing, e.g. observed in hypertrophic scars an keloids, and degenerative inflammatory and non-inflammatory diseases such as rheumatoid arthritis and osteoarthritis.

Antibodies according to the present invention, in particular, monoclonal antibodies according to the present invention, like the specific antibodies 203 E1 H5 and 234 H11 E8 as described herein are distinguished by detecting alpha-11 integrin subunit monospecifically both in biochemical and histological detecting systems. The antibodies are therefore suitable for the fast detection of the alpha-11 integrin subunit expression in very different samples.

The antibodies according to the present invention, e.g. in its humanized form, can functionally block integrin alpha-11 beta-1 mediated cell adhesion to collagen 1. Thus, these antibodies as described herein are suitable for the therapy of states of a disease like cancer, fibrosis, scleroderma, scarring in wound healing, e.g. observed in hypertrophic scars an keloids and degenerative inflammatory and non-inflammatory diseases such as rheumatoid arthritis and osteoarthritis.

For the production of the pharmaceutical composition or a medicine, the antibodies according to the present invention can be used alone or combined with common carriers, adjuvants and/or additives. The antibodies are suitable for the systemic, local, subcutaneous, intrathecal and topical application and for application by enema. In an embodiment, the antibody according to the present invention or the pharmaceutical composition according to the present invention suitable for the use according to the present invention are adapted to be administered intravenously, intranasally or intrabronchially. For this there can be applied solved and suitable solvents, preferably as aqueous solution, in the form of liposomes, as emulsion or in solid state, e.g. as powder or in the form of microcapsules.

Alternatively, the antibody according to the present invention or the pharmaceutical composition according to the present invention can be administered in a combined method of treatment with a different pharmaceutically active agent. Pharmaceutically active agents, that can be formulated with the antibodies according to the present invention, e.g. into a pharmaceutical according to the present invention or alternatively, can be administered in a combined method of treatment, can be for instance other antibodies thus providing a cocktail, or other active agents suitable for the treatment of the respective disease.

Further, active agents that can be formulated with the antibodies according to the present invention or alternatively can be administered in a combined method of treatment, especially in order to produce a therapeutically useful effect, depending on the disease state to be cured and are for instance commercially available active agents including antibiotics, anti-microbial products, antibacterial and antitumor agents or a mixture of two or more.

The antibodies according to the present invention as well as the pharmaceutical composition according to the present invention can be employed in an embodiment in the therapy of tumors, namely, cancer, alone or in combination with other therapeutics and forms of therapy, respectively, such as radiation. The dosage of the antibodies according to the present invention, e.g. present in the pharmaceutical composition according to the present invention will vary with the condition being treated in the recipient of the treatment, but will be in the range of 1 to about 100 mg for an adult patient preferably 1 to 10 mg usually administered daily for a certain period. That is, antibodies according to the present invention or the pharmaceutical composition according to the present invention may be administered on a regular basis for a period of time, like two, three, four, five, six days or one, two or three weeks or more, twice or more daily.

As noted, the pharmaceutical composition of the invention comprises a pharmaceutically acceptable excipient, carrier or diluent. These pharmaceutically acceptable carriers, excipients or diluents are determined in part by the particular composition being administered, as well as the particular method used to administer the composition. Accordingly, there is a wide variety of suitable formulations of pharmaceutical compositions of the present invention, see e.g. Remington's pharmaceutical sciences, 7th edition, 1989.

As noted before, the antibody according to the present invention is also useful for determining the alpha-11 integrin subunit in samples whereby said samples were fixed before by known methods, for example wherein the samples are acetone-fixed or formaldehyde-fixed before. That is, the use is particularly for staining alpha-11 integrin subunit tissues, preferably on cryopreserved or routinely fixed and paraffin embedded tissue. For this, the antibodies according to the present invention may be labelled appropriately, as described above, or employed in combination with the label antibodies directed against them or other reagents.

That is, the antibodies according to the present invention can be used diagnostically to monitor protein levels of the alpha-11 integrin subunit in tissue as part of clinical examination or clinical testing procedures, e.g. to determine the efficacy of a given treatment regimen or determining cancer as discussed below. Detection can be facilitated by coupling, e.g. physically linking, the antibody to a detectable substance. Examples of detectable substances include various enzymes, prosthetic groups, fluorescent materials, luminescent materials, bioluminescent materials and radioactive materials. Examples of suitable enzymes include horseradish peroxidase; alkaline phosphatase (-galactosidase, or acetylcholinesterase); examples of suitable prosthetic group complexes include streptavidin/biotin and avidin/biotin; examples of suitable fluorescent materials include fluorescein, fluorescein isothiocyanate, rhodamine, Cy-dyes, alexa fluor-dyes or brilliant violet dyes. Suitable radioactive materials include ¹²⁵I, ¹³¹I, ³⁵S or ³H while luminescent and bioluminescent materials include luminol as well as luciferase, luciferin and aequorin.

As noted above, detection may be also in combination with labelled secondary antibodies directed against the antibody according to the present invention. The skilled person is well known of suitable secondary antibodies which means that these antibodies are directed against the species from which the antibody according to the present invention is derived. Suitable examples in case of mouse monoclonal antibodies include gold anti mouse antibodies labelled with enzymes or dyes etc. which are commercially available.

The antibody are useful for detecting the alpha-11 integrin subunit expression by cultured cells or in cryopreserved tissue or tissues present in paraffin embedded samples. The present antibodies allow to detect routinely and specifically the alpha-11 integrin subunit in cryosections or paraffin embedded samples.

The antibody according to the present invention may, thus, be used in a method for determining the alpha-11 integrin subunit expression in a sample whereby said sample comprises cells and tissue, including the step of incubating the sample with the antibody according to the present invention and determining specific binding of said antibody by suitable means including biochemical and immunohistochemical detection. Detection means are described above including label and marker, said label and marker may be present with the antibody itself or may be present on secondary antibodies or secondary detection compounds containing the label or marker for detection accordingly.

In an embodiment of the method according to the present invention, the sample are tissue sections, in particular, human tissue sections. Further, in an embodiment of the present invention, the tissue sections are acetone, methanol or aldehyde fixed tissue sections, in particular, said tissue sections, or samples in general, are cryosections or paraffin embedded samples including paraffin embedded tissue sections or paraffin embedded cell sections.

The method according to the present invention may comprise further suitable steps for embedding the tissue and/or cells including paraffin embedding steps. The skilled person is well aware of the steps required for embedding the samples accordingly. In addition, the method according to the present invention may further comprise the necessary steps to treat the paraffin embedded samples to allow detection of antigens by antibodies accordingly.

Moreover, this method according to the present invention for detecting alpha-11 integrin subunit contain further steps of detection including immunohistochemical and immunohistological detection steps, like binding of secondary antibodies and staining of the sections when using enzyme based detection systems or microscopy etc. when using fluorescence based or chromophore based systems.

The method according to the present invention are useful for detecting various types of cancer. In particular, the antibodies according to the present invention as well as the method according to the present invention allow to identify fibroblastoid expression in keratin-negative, vimentin-positive, non-vessel associated stroma of invasive breast carcinoma, ovary adenocarcinoma, skin carcinoma and pancreas adenocarcinoma. In general, the antibodies according to the present invention may serve as a biomarker for CAFs in carcinoma-type tumor characterized by a desmoplastic stroma.

As outlined above, the present invention relates further to a pharmaceutical composition comprising the antibody according to the present invention. In an embodiment, the pharmaceutical composition according to the present invention comprises both, the 203 E1 H5 antibody and the 234 H11 E8 antibody or the humanized antibodies stemming from said antibodies.

In another embodiment, the antibodies present in the pharmaceutical composition are humanized antibodies as described herein.

In the pharmaceutical composition, the antibodies according to the present invention may be present in form of antibody drug conjugates (ADC). ADC have revolutionized the field of cancer chemotherapy. ADC utilize the antibody moiety to specifically bind tumor associated target antigens and deliver a highly potent cytotoxic agent. Thus, an extremely efficient class of anti-cancer drugs can be supplied using the synergistic combination of the antibody conjugated to the chemotherapeutic. A review of ADC is provided by Peters C. and Brown S., Biosci. Rep., 2015, 35, art:e00225, doi:10.1042/BSR20150089, which is incorporated herein by reference.

That is, the antibody according to the present invention or the pharmaceutical composition according to the present invention are particularly useful in treating or preventing cancer. For example, the antibodies according to the present invention are in form of antibody drug conjugates. In an embodiment, the cancer is selected from breast cancer, ovary adenocarcinoma, skin carcinoma, pancreas adenocarcinoma and non-small-cell lung adenocarcinoma.

Further, the antibody according to the present invention or the pharmaceutical composition according to the present invention are useful in treating or preventing fibrosis, or scleroderma as well as degenerative inflammatory and non-inflammatory diseases, such as rheumatoid arthritis and osteoarthritis.

In addition, it has been recognized that the antibody according to the present invention or the pharmaceutical composition according to the present invention might be useful in preventing excessive scarring, e.g. observed in hypertrophic scars an keloids.

In a further embodiment of the present invention, the present invention relates to a kit for preventing or treating cancer, fibrosis, scleroderma or for preventing excessive scarring in wound healing, e.g. observed in hypertrophic scars an keloids as well as for treating degenerative inflammatory and non-inflammatory diseases, such as rheumatoid arthritis and osteoarthritis. Said kit comprises the antibody according to the present invention or the pharmaceutical composition according to the present invention and, optionally, a further therapeutic agent for treating said disease. That is, said kit provides for a combination therapy of the referenced diseases.

Another embodiment of the present invention relates to a method for treating the mentioned diseases, namely for treating cancer like breast cancer, ovary adenocarcinoma, skin carcinoma, pancreas adenocarcinoma and non-small-cell lung adenocarcinoma as well as treating or preventing fibrosis, scleroderma and degenerative inflammatory and non-inflammatory diseases such as rheumatoid arthritis and osteoarthritis as well as a method for preventing excessive scarring in wound healing, e.g. observed in hypertrophic scars an keloids. Said method comprises the step of administering the antibody according to the present invention or the pharmaceutical composition according to the present invention to a subject in need thereof.

In an embodiment, the antibody administered to the subject in need thereof is a humanized antibody, in particular, a humanized antibody stemming from the 203 E1 H5 antibody or the 234 H11 E8 antibody as described herein. In another embodiment, the subject in need thereof is a mammal, in particular, a human.

Further, the present invention relates to the use of the antibodies according to the present invention in separating, purifying or isolating mesenchymal stem cells. Moreover, the present invention relates to a method of separating, purifying or isolating mesenchymal stem cells with the antibodies according to the present invention. These separated, isolated or purified mesenchymal stem cells may be used in various applications including cartilage healing, production of artificial tissue, etc. For example in case of cartilage production, the unwanted fibroblasts may be separated from the cell culture leaving the cartilage cells, thus, enabling in vitro generation of cartilage tissue.

As noted above, the skilled person is well aware of suitable ways of administration of said compounds.

### Examples

The following examples have been included to illustrate modes of the present disclosed subject matter. In light of the present disclosure and the general level of the skilled in the art, those of skilled in the art will appreciate that the following examples are intended to be exemplary only and that numerus changes and modifications can be applied without departing from the scope of the present disclosed subject matter.

### Material and Methods

**Cell lines -** C2C12 cells stably expressing human α11 integrin or human α2 integrin subunits (C2C12-α11 and C2C12-α2, respectively [6]) were cultured in DMEM medium and 10% fetal bovine serum (FBS; Gibco) supplemented with antibiotics.

### Generation of Mab specific to integrin α11 chain

MAbs were produced using established procedures. NT-HRM mice (nanoTools Antikoerpertechnik, Germany) were immunized with human α11β1 integrin (R&D Systems), boosted twice and cell fusion was performed on day 68. Luminex beads coated with α11β1 integrin were used to screen α11 binders. Supernatants from positive clones were tested in FACS using C2C12- α11 cells as positive control and wildtype C2C12 (do not express human integrins), C2C12 -α2 cells and A431 cells (do express human integrin β1, but not α11) as negative controls. Positive clones were further characterized and finally subcloned by limited dilution.

### Immunostaining

Cells were washed in PBS and fixed with ice cold methanol 10 min at -20C. Cells were blocked with 5% BSA/PBS containing 0.1% Triton X-100 for 1 hour at RT. Next, cover slips were incubated with primary antibody, 203 E1 H5 or 234 H11 E8, in 5% BSA/PBS with 0.1 % Triton X-100 for 1 hour at 37°C. Cells were then washed with 0.05% Tween-20/PBS and incubated with Alexa fluor® 594 goat anti-mouse IgG (1:400, Jackson ImmunoResearch) for 1 hour at RT. Later, coverslips were incubated with DAPI (0.25 µg/ml, Invitrogen) and mounted with ProLong Diamond Antifade mounting medium (Thermo Scientific). Cells were visualized under a Zeiss Axioscope fluorescence microscope and pictures were acquired with a digital AxioCam MRm camera.

### Flow cytometry

Cells were harvested in PBS (without Ca²⁺ and Mg²⁺) and accutase® (Biochrom) and washed in PBS (without Ca²⁺ and Mg²⁺). 40µl of integrin α11 antibody supernatants (diluted 1:8 with Fluorescence activated cell sorting (FACS) buffer (PBS/0.2 mM Ca²⁺, 0.1% BSA and 0.1% Poloxamer 188)) were mixed with 10⁵ cells and incubated 1 hour at 4°C. Cells were then washed once with FACS buffer and incubated 1 hour at 4°C with 50µl of PE labelled goat anti-mouse (diluted 1:400 in FACS buffer). Cells were washed once with FACS buffer and the samples analyzed by flow cytometry using Intellicyt iQue.

### Immunoprecipitation

Subconfluent C2C12-α11 cells were labelled overnight in DMEM without methionine and cysteine supplemented with 2% FCS and 25 µCi/ml ³⁵S ProMix (GE Healthcare, Buckinghamshire, UK). Following overnight culture, medium was removed and cells were lysed using ice-cold solubilization buffer (1 % Triton X-100, 0.15 M NaCl, 10 mM Tris-HCl pH 7.4, 1 mM MgCl₂, 1 mM CaCl₂) containing a protease inhibitor cocktail (complete mini EDTA-free, Roche Diagnostics). Cell lysates were centrifuged for 10 min at 4°C at 13000 rpm. The supernatant was pre-cleared by incubation with 100 µg/ml of preimmune IgG and protein A-Sepharose CL 4B (GE Healthcare) at 4°C overnight. In the next step, samples were incubated with 100 µg/ml of rabbit polyclonal anti-human α11 antibody [7] or mouse mAb 203E1 H5 or mAb 234H11E8 anti-human integrin α11 hybridoma supernatants. The precipitates were washed three times with high salt buffer (1% Triton X-100, 0.5 M NaCl, 10 mM Tris-HCl pH 7.4, 1 mM MgCl₂, 1 mM CaCl₂) and three times in a physiological salt buffer (0.1% Triton X-100, 0.15 M NaCl, 10 mM Tris-HCl pH 7.4, 1 mM MgCl₂, 1 mM CaCl₂) before solubilization in sodium dodecyl sulphate (SDS) -sample buffer with reducing agents. Proteins were separated by 6% SDS - poly acrylamide gel electrophoresis (PAGE) and processed for autoradiography.

### Cell attachment assay

Prior to cell attachment, a 24-well plate was coated with either 2% BSA as negative control or with 100 µg/ml of type I collagen and incubated 1 hr at 37°C. After three washings with PBS, coatings were blocked with 2% BSA for 1 hr at 37°C, followed by three more washings with PBS. The C2C12-α11 cells were detached from culture plate and washed three times in plain DMEM before being incubated on ice for 30 min with or without the mouse monoclonal antibodies, namely, 203 E1 H5 and 234 H11E8 anti-human integrin alpha-11 (10 µg/ml) or integrin beta-1 Ha2/5 (BD Biosciences; 10 µg/ml, used as positive control for cell adhesion blocking). Cells (2x10⁵/well, in 24-w plates) were then seeded on the previous coated plate and incubated 40 min at 37°C. After incubation, non-attached cells were removed by successive shakings and washings; the remaining cells were fixed in 1.1% glutaraldehyde for 15 min and stained in crystal violet for 20 min. After washing, crystal violet was released using 10% acetic acid and absorbance was measured at 560nm.

### Collagen gel contraction assay:

Collagen gel contraction was performed as described previously [8]. Briefly, each ml contained: 500 µl of 2 X DMEM containing 2x10⁵ cells per ml, 100 µl 0.2M HEPES (Sigma) ph 8.0 and 400 µl collagen type I (PureCol, Advanced BioMatrix). 400 µl of this mixture was added into each well of a 24-well plate and were allowed to polymerize (approximately 90 min) at 37°C in order to obtain floating conditions, gels were poured into wells that had been previously coated overnight with 2% BSA in sterile PBS. Once the cell-containing collagen mixture had polymerized, 400 µl of DMEM supplemented with 0.5 % FCS was added. For testing the effect of 203 E1 H5 an 234 H11 E8 on collagen remodelling, the antibodies were included with the DMEM and FCS at this step.

### Results

The immunological characterization as shown in figures 1 and 2 demonstrates that both antibodies namely 203 E1 H5 and 234 H11 E8 are specific for alpha-11 integrin subunit. Further, it is demonstrated in figure 1e and 2e, respectively, that integrin alpha-11 staining in cell focal adhesion can be determined.

Moreover, by way of cell adhesion and collagen gel contraction experiments it is demonstrated that both integrin alpha-11 antibodies, 203 E1 H5 and 234 H11 E8 block functionally integrin alpha-11 beta-1 mediated cell adhesion to collagen I. In contrast, said antibodies do not affect integrin alpha-2 beta-1 mediated cell adhesion to collagen I, see figure 3. Further, both antibodies were blocked functionally integrin alpha-11 beta-1 mediated collagen reorganization. Thus, these antibodies represent suitable means for either detecting the alpha-11 integrin subunit as well as for functionally blocking activity of the alpha-11 beta-1 integrin. Hence, the antibodies according to the present invention represent a suitable tool for the treatment as described herein.

### References

1. Zhu, C.Q., et al., Proc Natl Acad Sci USA, 2007. 104(28): p. 11754-9.
2. Navab, R., et al., Oncogene, 2016. 35(15): p. 1899-908.
3. Parajuli, H., et al., J Oral Pathol Med, 2017. 46(4): p. 267-275.
4. Westcott, J.M., et al., J Clin Invest, 2015. 125(5): p. 1927-43.
5. Sun KH, C.Y., Reed NI, Sheppard D, Am J Physiol Lung Cell Mol Physiol., 2016. doi: 10.1152/ajplung.00350.2015**. [Epub ahead of print].**
6. Tiger, C.F., et al., Dev Biol, 2001. 237(1): p. 116-29.
7. Veiling, T., et al., J Biol Chem, 1999. 274(36): p. 25735-25742.
8. Schulz, J.N., et al., J Invest Dermatol, 2015.135(5): p. 1435-44.

## Claims

1. An antibody i) that binds to the same epitope of the alpha-11 integrin subunit as bound by 203 E1 H5 produced by the hybridoma deposited as DSM ACC3318 or ii) that binds the same epitope of the alpha-11 integrin subunit as bound by 234 H11 E8 produced by the hybridoma deposited as DSM ACC3319.

2. The antibody of claim 1 wherein the antibody is either 203 E1 H5 produced by the hybridoma deposited as DSM ACC3318 or 234 H11 E8 produced by the hybridoma deposited as DSM ACC3319.

3. The antibody of claim 1 or 2, wherein the antibody is a humanized antibody.

4. The humanized antibody according to claim 3 that specifically binds to the alpha-11 integrin subunit, comprising the heavy and light chain complementarity determining regions (CDR) of the antibody as defined in claim 1 or 2.

5. The humanized antibody according to claim 4, wherein the CDR are CDR of the antibody produced either by the hybridoma deposited as DSM ACC3318 or produced by the hybridoma deposited as DSM ACC3319.

6. The antibody according to any one of the preceding claims wherein the antibody is selected from the group consisting of a scFv, a Fab, and a (Fab')₂.

7. A pharmaceutical composition comprising the antibody according to any one of claims 1 to 6 and, optionally, a pharmaceutically acceptable excipient, carrier or diluent.

8. The pharmaceutical composition according to claim 7 comprising both, the 203 E1 H5 antibody and 234 H11 E8 antibody or the humanized antibodies stemming therefrom according to any one of claims 1 to 6.

9. The pharmaceutical composition according to claims 7 to 8 wherein the antibody is present in form of an antibody-drug conjugate.

10. The antibody of any one of claims 1 to 6 or the pharmaceutical composition according to claim 7 or 9 for use in treating or preventing fibrosis or scleroderma.

11. The antibody according to any one of claims 1 to 6 or the pharmaceutical composition according to claim 7 or 9 for use in treating or preventing cancer.

12. The antibody or the pharmaceutical composition for use according to claim 11 wherein the cancer is selected from breast carcinoma, ovary adenocarcinoma, skin carcinoma, pancreas adenocarcinoma and non-small cell lung adenocarcinoma.

13. The antibody of any one of claims 1 to 6 or the pharmaceutical composition according to claim 7 or 9 for use in preventing excessive scarring in wound healing.

14. The antibody of any one of claims 1 to 6 or the pharmaceutical composition according to claim 7 or 9 for the use according to any one of claims 10 to 13 adapted to be administered intravenously, intranasally, or intrabronchially.

15. A kit for treating or preventing cancer, fibrosis, scleroderma, or preventing excessive scaring in wound healing comprising the antibody of any one of claims 1 to 6 or the pharmaceutical composition according to claim 7 or 9 and, optionally, a further therapeutic agent for treating said disease.
